(19) Europäisches Patentamt
European Patent Office
Office européen des brevets

(11) Publication number: 0 122 804 B1

# (12) EUROPEAN PATENT SPECIFICATION

(45) Date of publication of patent specification: 10.12.86

(21) Application number: 84302586.7

(22) Date of filing: 16.04.84

(51) Int. Cl.[4]: C 07 C 51/285, C 07 C 53/126, C 07 C 55/14, C 07 C 63/06

## (54) Process for preparing carboxylic acids from olefins or vicinal dihydroxy compounds.

(30) Priority: 15.04.83 IT 2060483

(43) Date of publication of application: 24.10.84 Bulletin 84/43

(45) Publication of the grant of the patent: 10.12.86 Bulletin 86/50

(84) Designated Contracting States: BE CH DE FR GB LI NL

(56) References cited:
EP-A-0 109 273
GB-A-1 324 763
US-A-3 855 257

(73) Proprietor: Montedison S.p.A.
31, Foro Buonaparte
I-20121 Milan (IT)

(72) Inventor: Venturello, Carlo
135, Via XXIII Marzo
Novara (IT)
Inventor: Ricci, Marco
5, Via Brescia
Novara (IT)

(74) Representative: Whalley, Kevin et al
Marks & Clerk 57/60 Lincoln's Inn Fields
London WC2A 3LS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 122 804 B1

**Description**

The present invention relates to a process for preparing carboxylic acids from olefins or vicinal dihydroxy compounds.

More particularly, this invention relates to a process according to which carboxylic acids are prepared by the oxidative scission of olefins or of their corresponding vicinal dihydroxy compounds by means of $H_2O_2$, in a biphasic aqueous liquid/organic liquid system, in the presence of suitable catalysts.

Various processes are known for the preparation of carboxylic acids. Amongst the various different procedures followed, the oxidative scission of olefinic hydrocarbons represents, due to the wide availability of the raw materials, a particularly attractive way.

Olefinic hydrocarbons may be oxidized to carboxylic acids by the use of various oxidizers, such as $KMnO_4$, $K_2Cr_2O_7$ and $RuO_4$. These processes are, however, of little practical interest because of the high cost of the oxidizers used as well as because of their toxicity (particularly with regard to $RuO_4$) and the serious problem involved in their disposal or their recovery at the end of the reaction.

In the case of ruthenium, in order to obviate in part the above drawbacks, there have been suggested a number of oxidation processes of olefins to carboxylic acids, based on the use of a ruthenium compound (for instance $RuCl_3$) in catalytic quantities, combined with the use of various oxidizers such as NaOCl, $NaIO_4$, $Ce^{IV}$ salts, $CrO_3$ together with $HNO_3$ and, lastly, organic peracids (for instance peracetic acid), capable of re-oxidizing to the maximum valency the reduced catalyst.

However, in the case of these catalytic procedures, there also occur difficulties of an economic and/or environmental character. For example, in the case of the use of $NaIO_4$, $NaClO_4$ or of $Ce^{IV}$ salts as primary oxidizers, in order to make the process technically feasible, it becomes necessary to ensure their complete recovery, an operation that is very difficult to realize in practice.

To these difficulties there must be added the problems connected with the purification of the effluent waters of the process. Likewise, in the case of the use of organic peracids, even if there is no problem of polluting effluent waters, there still exist the problems of cost of recovery or of use of the acid coming from the peracid.

On the other hand, the catalytic oxidation of olefins to carboxylic acids using aqueous $H_2O_2$ as a primary oxidizer may offer undoubted advantages with respect to the above-mentioned methods, due to the relatively modest cost of the oxidizer and to the absence of a reduction product to be disposed of. However, this approach has not been the object of particular interest because of the poor efficiency of $H_2O_2$ as an oxidizer in the above said reaction. The few examples reported in the patent literature concern only the oxidation of particularly reactive olefins (that is cycloolefins) to carboxylic acids; these give rather unsatisfactory yields in acids and are always characterized by the use, as catalysts, of particularly expensive and toxic metal oxides such as $OsO_4$ and $Re_2O_7$.

It has also been suggested to prepare carboxylic acids from vicinal dihydroxy compounds using various oxidizers, for example $KMnO_4$ or peracetic acid associated to $RuCl_3$ as catalyst; however, there occur drawbacks of the same type as those described for the processes that start from olefins.

U.S. Patent No. US—A—3855257 discloses a process for preparing carboxylic acids comprising oxidizing an internal vicinal glycol with oxygen in the presence of a catalytic amount of a mixture comprising a cobalt (II) salt of an organic acid and a compound selected from the group consisting of peroxidized tungstic acid, peroxidized tungstic oxide, peroxidized molybdic acid and peroxidized molybdic oxide in a polar, aprotic solvent.

British Patent No. GB—A—1324763 discloses a method for conducting heterogenous ionic organic reactions wherein the reactants are in a plurality of immiscible liquid phases with one of the reactants comprising an olefin being located substantially entirely in a different phase from the contiguous phase in which a second reactant comprising an oxidising agent is located; which method comprises adding to one or both of the contiguous phases of the heterogeneous system, an organic quaternary salt having the formula

$$(R_1R_2R_3R_4M)^+X^-$$

where M is a pentavalent ion of an element consisting of nitrogen, phosphorus, arsenic, antimony or bismuth, $R_1$, $R_2$, $R_3$ and $R_4$ are hydrocarbon radicals wherein one of the hydrocarbon radicals may be substituted by a further quaternary group the total number of carbon atoms being from 18 to 70, and $X^-$ is an anion which will dissociate from the cation

$$(R_1R_2R_3R_4M)^+$$

in an aqueous environment, said organic quaternary salt being substantially more soluble in the less polar of said reactant-containing phases than in the other of said reactant-containing phases and a metal compound, the metal consisting of osmium, ruthenium, molybdenum, tungsten, selenium, vanadium, chromium, titanium, cerium, nickel, manganese, cobalt, platinum, iron, lead or palladium.

The present invention aims to provide a new and convenient catalytic process for the preparation of monocarboxylic and bicarboxylic acids starting from olefins or from their corresponding vicinal dihydroxy compounds, that is free of the drawbacks and limitations of the above described processes and that uses aqueous $H_2O_2$ as an oxidizing agent.

The present invention provides a process for

the preparation of a monocarboxylic or bicarboxylic acid, by oxidative scission of an olefin or the corresponding vicinal dihydroxy compound. This process is characterized in that an olefin of the formula

$$R_1—CH=CH—R_2 \text{ or } R_2—CH=CH_2$$

or its corresponding dihydroxy compound of formula

$$R_1—CHOH—CHOH—R_2 \text{ or } R_1—CHOH—CH_2OH$$

(wherein $R_1$ and $R_2$ may be the same as or different from each other and are optionally substituted with one or more groups inert under the reaction conditions, and represent hydrocarbon groups such as alkyls having up to 30 carbon atoms; cycloalkyls, optionally branched, having from 3 to 12 carbon atoms; aryls and alkylaryls having from 6 to 12 carbon atoms; and wherein, moreover, $R_1$ and $R_2$ may be bound to each other so as to form an alkenyl or alkyl cycle having up to 12 carbon atoms) is reacted, under vigorous stirring, with $H_2O_2$ at a temperature from 0°C to 120°C and under a pressure from 1 to 100 atmospheres; there are used an aqueous phase containing $H_2O_2$ and an organic phase consisting of a solvent, the olefin or its corresponding vicinal dihydroxy compound, and a catalyst; in the case of an olefin, the catalyst is a composition of the formula:

$Q_3XW_4O_{24-2n}$ wherein:

Q represents an onium $(R_5R_6R_7R_8M)^+$ cation in which M is selected from N, P, As and Sb, and $R_5$, $R_6$, $R_7$ and $R_8$, which may be the same as or different from each other, represent hydrogen atoms or hydrocarbon groups having in total from 20 to 70 carbon atoms;
X is P or As; and
n is 0, 1 or 2;

and in the case of a vicinal dihydroxy compound, the catalyst is selected from catalysts of the composition

$$Q_3XW_4O_{24-2n}$$

and from catalysts obtained by reaction of tungstic acid or an alkali tungstate with hydrogen peroxide and an onium $Q^+Y^-$ salt (wherein $Y^-$ is an organic anion) at a pH below 2.

In the following are represented the oxidative scission reactions of the olefins and of their corresponding vicinal dihydroxy compounds:

$R_1—CH=CH—R_2+4H_2O_2⟶$

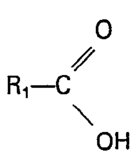 + 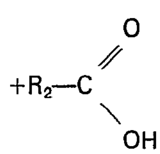 $+4H_2O$ (1)

$R_1—CH=CH_2+5H_2O_2⟶$

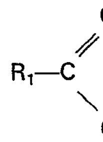 $+CO_2+6H_2O$ (2)

$R_1—CHOH—CHOH—R_2+3H_2O_2⟶$

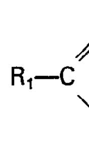 + 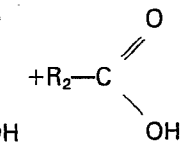 $+4H_2O$ (3)

$R_1—CHOH—CH_2OH+4H_2O_2⟶$

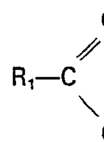 $+CO_2+6H_2O$ (4)

As can be seen from the above, reactions (1) and (3) produce two different carboxylic acids:

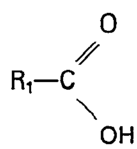 and 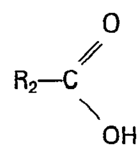 ;

however when $R_1$ is the same as $R_2$, only one acid is obtained. When the olefin

$$R_1—CH=CH_2$$

or the vicinal dihydroxy compound

$$R_1—CHOH—CH_2OH$$

are terminal, there are formed

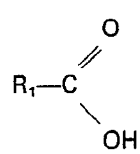

and formic acid, which may be oxidized in the reaction medium to $CO_2$; in the case of complete oxidation of

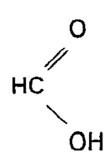

to $CO_2$, reactions (2) and (4) occur.

If $R_1$ and $R_2$ are bound together in such a way as to form a cycle, there is obtained a bicarboxylic acid schematically represented by the formula:

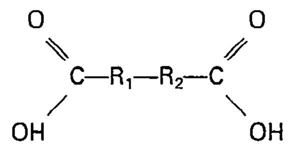

The compositions of formula $Q_3XW_4O_{24-2n}$ and their manner of preparation are described in European Patent Application No. 83306883, filed on 10.11.1983.

These catalysts may be prepared in the following way: Tungstic acid or an alkali tungstate and phosphoric acid or an alkali phosphate (or a corresponding arsenic compound) are first made to react in an aqueous acid phase with $H_2O_2$, at a temperature from 20° to 80°C. The acid aqueous phase preferably has a pH value below 2; in order to reach such a value the pH is appropriately adjusted with a mineral acid (for example $H_2SO_4$ or HCl). Subsequently there is added, preferably at room temperature, an onium salt contained in an organic solvent immiscible with water (for instance dichloroethane or benzene). The onium $Q^+Y^-$ salt consists of a $Q^+$ cation as already defined and of an inorganic $Y^-$ anion that is stable under reaction conditions, such as for example $Cl^-$, $HSO_4^-$ or $NO_3^-$. The stirring of the biphasic mixture is carried on for 15—30 minutes.

The molar ratios between the reactants are usually the following: for each gram atom of X (P or As), there are used at least 4 gram atoms of W and up to 2 mols of onium salt; as far as the $H_2O_2$ is concerned, there suffice from 2.5 to 6 mols of $H_2O_2$ per each gram atom of W.

If the product thus formed is in the solid state, it is directly separated from the biphasic mixture, for example by filtering. In the contrary case, there will be separated the organic phase which will be filtered and evaporated under vacuum at from 40°C to 50°C, thereby obtaining the catalyst in the form of a solid or a thick oil.

Amongst the catalysts of formula $Q_2XW_4O_{24-2n}$ there are preferred those in which the radicals $R_5$, $R_6$, $R_7$ and $R_8$ of the onium Q cation have a total of from 25 to 40 carbon atoms.

As far as the second type of catalyst is concerned, that obtained by the reaction of tungstic acid or of an alkali tungstate with hydrogen peroxide and with an onium $Q^+Y^-$ salt (wherein $Y^-$ is an inorganic anion), at a pH below 2, this catalyst may be prepared in the following way: tungstic acid or an alkali salt thereof is suspended or dissolved in water in the presence of $H_2O_2$ at a temperature from 20° to 80°C. The pH of this solution or suspension is, if required, adjusted by means of a mineral acid (for example $H_2SO_4$, or HCl), until a pH value below 2 is obtained (preferably a value equal to or greater than 0 but below 2). Then, under stirring, there is admixed, preferably at room temperature, an onium $Q^+Y^-$ salt (wherein $Y^-$ is for example $Cl^-$, $HSO_4^-$ or $NO_3^-$) dissolved in an organic water immiscible solvent (for instance benzene or dichloroethane).

The ratios between the reactants are usually the following: for each gram atom of W, there are used from 3 to 5 mols of $H_2O_2$ and from 0.4 to 1 mol of onium salt.

The stirring of the biphasic mixture is carried on for 15—30 minutes. At the end the organic phase is separated, filtered and evaporated under vacuum at 40°—50°C, thereby obtaining a thick yellow oil that is the desired catalyst.

The second type of catalyst may also be prepared *in situ* in the reaction medium. For this purpose, into the reactor are placed the tungstic acid or an alkali tungstate, the hydrogen peroxide, the onium salt, the vicinal dihydroxy compound, the solvent and, if required, a mineral acid ($H_2SO_4$ or HCl) in an amount sufficient to bring the pH of the aqueous phase to a value below 2.

In place of the tungstic acid or of the tungstate, there may be used tungsten compounds capable of forming tungstate ions in the reaction medium, e.g. $WO_2$, $W_2O_5$, $WO_3$, $WS_2$, $WS_3$, $WCl_6$, $WOCl_4$ and $W(CO)_6$.

Also as far as the second type of catalyst is concerned, there are preferably used onium salts in which radicals $R_5$, $R_6$, $R_7$ and $R_8$ of the onium Q cation have in total from 25 to 40 carbon atoms.

In the preparation of carboxylic acids starting from vicinal dihydroxy compounds, the catalysts of formula

$$Q_3XW_4O_{24-2n}$$

in general ensure better yields than those obtainable with the second type of catalyst, and are therefore the preferred ones.

As solvents for the organic phase there are used inert solvents that are substantially immiscible with the aqueous phase. For example, there may be used: 1) aromatic hydrocarbons such as benzene, toluene and xylenes; 2) chlorinated hydrocarbons such as dichloromethane, trichloromethane, chloroethane, chloropropanes, dichloroethanes, trichloroethanes, tetrachloroethanes, dichloropropanes, trichloropropanes, tetrachloropropanes, and chlorobenzene; and 3) alkyl esters such as ethyl acetate.

There may also be used suitable mixtures of the above-mentioned solvents.

The $R_1$ and $R_2$ groups of the starting olefins and of the starting vicinal dihydroxy compounds may optionally contain one or more groups (usually from 1 to 4) inert under the reaction conditions. These inert groups are, for instance, hydroxyl, chloride, fluoride, nitro, an alkoxy $OR_9$ group (wherein $R_9$ is a hydrocarbon group having up to 10 carbon atoms), a ketone group, a carboxylic group, an ester $COOR_{10}$ group (wherein $R_{10}$ is a hydrocarbon group having up to 10 carbon atoms), an amidic group, and/or a nitrile group.

Amongst the unsaturated aliphatic and cycloaliphatic hydrocarbons oxidizable to carboxylic acids by the process of the present invention, there may be mentioned: 1-hexene, 1-octene, 1-decene, 1-dodecene, 1-tetradecene, 1-nonadecene, 1-eicosene, 2-hexene, 2-octene, 4-octene, cyclopentene, cyclohexene, cycloheptene, cyclododecene, and distillation fractions of olefins, obtained by fractional distillation, derived from cracking processes, being commercially available as mixtures of olefins having 6 to 10, 10 to 15 or 15 to 18 carbon atoms, respectively, on the basis of their boiling points.

Amongst unsaturated aromatic hydrocarbons there may be mentioned; styrene, stilbenes and vinylnaphthalene. Amongst the olefinic compounds containing functional groups that are inert under reaction conditions, there may be mentioned: undecylenic acid, oleic acid and elaidic acid.

Amongst the vicinal dihydroxy compounds oxidizable to carboxylic acids by the process of the present invention, there may be mentioned: the vicinal dihydroxy compounds of the above-mentioned unsaturated hydrocarbons, for example 1,2-dodecanediol, 1,2 - octanediol, 1,2 - cyclohexanediol, 1,2 - cycloheptanediol, 1 - phenyl - 1,2 - ethanediol, 1,2 - hexanediol, 4,5 - octanediol, 6,7 - dodecanediol, 1,2 - diphenyl - 1,2 - ethanediol and 1,2 - cyclopentanediol.

As already mentioned, the reaction between the olefin of the vicinal dihydroxy compound and the $H_2O_2$, in the presence of the catalyst, occurs under vigorous stirring: by the term "vigorous stirring" is meant such stirring as will allow a continuous mixing through of the organic phase with the aqueous phase.

The reaction is carried out with the so-called phase-transfer technique in a biphasic aqueous liquid/organic liquid consisting of:

a) an organic phase containing a solvent, the olefin or the vicinal dihydroxy compound, and the catalyst;

b) an aqueous phase containing the $H_2O_2$.

The operational temperature is practically determined by the reactivity and by the nature of the olefin or of the vicinal dihydroxy compound and by the stability of the hydrogen peroxide and of the catalyst used. Generally the reaction is conducted at a temperature from 20°C to 120°C and, more preferably, at a temperature from 40° to 90°C. Starting from olefins, the operational pressure is generally atmospheric pressure. However, in the case of low-boiling olefins, it is necessary to operate at a pressure that is sufficient (up to 100 atmospheres) to maintain the olefin in the liquid state.

Starting from vicinal dihydroxy compounds, the operational pressure is usually substantially atmospheric pressure.

The reactants ($H_2O_2$ and olefin or vicinal dihydroxy compound) are used substantially according to the molar ratios corresponding to the stoichiometry of the reactions from (1) to (4). However, it is preferred to use a moderate excess (for example of about 10%) of $H_2O_2$ with respect to the stoichiometry.

The catalyst is preferably used in an amount from 0.01 and 1 gram atom of W per one mol of substrate (olefin or vicinal dihydroxy compound), more preferably from about 0.05 to about 0.15 gram atom of W per mol of substrate.

The concentration of the olefin or of the vicinal dihydroxy compound in the organic phase is in general from 5% to 95% by weight, more preferably from about 20% to about 50% by weight.

The concentration of the $H_2O_2$ in the aqueous phase is in general from 1% to 70% by weight, more preferably from about 10% to about 50% by weight.

In some cases, in order to obtain better yields, it has proved convenient to add to the reaction mixture small quantities of p-tert.butylphenol as an inhibitor of radical reactions.

The duration of the reaction depends on the nature and on the quantity of catalyst and on the type of solvent and of olefin or vicinal dihydroxy compound used. In general a period of time from 4 to 15 hours is sufficient for completing the reaction.

At the end of the reaction the acid or the acids may be recovered from the reaction medium by utilizing conventional techniques.

The organic acids obtained by the process of the present invention find a variety of applications. For example, adipic acid is used in the production of polyamides, while esters of azelaic and pelargonic acids are used as plasticizers.

Using starting olefins of the type

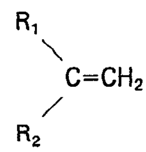

or vicinal dihydroxy compounds of the type

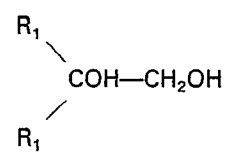

there may be formed ketones instead of acids.

The invention will be further described with reference to the following illustrative Examples.

Example 1

Into a 100 ml flask, provided with a reflux coolant and a magnetic stirrer, there were loaded: 1.4 g of the composition

$$[(C_8H_{17})_3NCH_3]_3PW_4O_{22}$$

(equal to about 2.5 mmols of W), 5 ml of 1,2-dichloroethane, 2.24 g (20 mmols) of 1-octene, 9.35 ml of $H_2O_2$ at a concentration of 400 g/lt (110 mmols) and about 2 mg of p-(ter-butyl)-phenol. The resulting mixture was brought to 80°C, under vigorous stirring, and then maintained at that temperature for 6.5 hours.

At the end of this time the phases were separated. The aqueous phase was extracted three times with 1,2-dichloroethane in order to extract the acids dissolved in water, after which the extract was added to the organic phase.

The organic phase was extracted with $Na_2CO_3$ in a 10% concentration (3×10 ml), after which it was eluted with n-hexane on an ion exchange resin of the sulphonic type in an acid form (Dowex 50 W, 50—100 mesh) in order to free the acids from their quaternary ammonium salts, and the eluted substance containing the acids was extracted again with 10% $Na_2CO_3$.

The basic aqueous extracts thus obtained were then combined and acidified with 10% HCl. The resulting mixture was then extracted with n-hexane (3×20 ml). By evaporation of the hexane there were obtained 1.73 g of $C_6$—$C_8$ acids of which 94.7% (measured by gas chromatography) consisted of enanthic ($C_7$) acid. The yield in enanthic acid amounted to 63% (calculated on the olefin).

Example 2

Example 1 was repeated, but using composition

$$[(C_8H_{17})_3NCH_3]_3AsW_4O_{20}$$

(1.4 g; 2.5 mmols). There were obtained 1.78 g of $C_6$—$C_8$ acids, 94.6% of which consisted of enanthic acid ($C_7$). The yield in enanthic acid amounted to 65%.

Example 3

Example 1 was repeated, but in the absence of p-(ter-butyl)-phenol, and using styrene (2.08 g, 20 mmols) instead of 1-octene. After acidification of the aqueous basic extracts with HCl in a 10% concentration, the obtained crystalline solid was filtered, washed with $H_2O$, dried on $P_2O_5$ and then dissolved in ether. By evaporation of the preliminarily filtered etheric solution, there were obtained 1.83 g of benzoic acid with a 97.7% titre, with a yield of 73%.

Example 4

Example 1 was repeated, but using oleic acid (5.65 g; 20 mmols) instead of 1-octene, and using 7.50 ml instead of 9.35 ml of $H_2O_2$ at a concentration of 400 g/lt (88.2 mmols) and by reducing the reaction time to 5 hours. At the end of this time, the reaction mixture was allowed to rest overnight in a refrigerator (0°C—5°C).

After filtering of the solid that had formed and after successive separation of the phases, there was followed the procedure as described in example 1. The solid, gathered by filtering, was added to the residual oil obtained by evaporation of the n-hexane, and the resulting mixture was eluted on a silicon column (70—230 mesh; eluent: acetone/n-hexane 1:1) gathering the fractions having $R_f$=0.5—0.9.

There were obtained 5.48 g of substance consisting of 56.7% of azelaic acid (yield: 83%), 37.9% of palargonic acid (yield: 66%), and 1.8% of caprilic ($C_8$) acid.

Example 5

Example 1 was repeated, but using 1-dodecene (3.36 g, 20 mmols) instead of 1-octene. The residual oil, obtained by evaporation of the n-hexane, was eluted in a silicon column (70—230 mesh; eluent: ether/n-hexane 1:1) gathering the fractions having $R_f$ of about 0.5—0.6.

By evaporation of the solvent there were obtained 2.04 g of $C_8$—$C_{11}$ acids of which 90.1% were undecanoic acid ($C_{11}$). The yield in undecanoic acid amounted to 49%.

Example 6

Into a 100 ml flask, provided with a reflux coolant and a magnetic stirrer, were loaded 1.4 g (equal to about 2.5 mmols of W) of the catalytic composition of example 1, 10 ml of 1,2-dichloroethane, 4.26 g (52 mmols) of cyclohexene and 19.55 ml of $H_2O_2$ at a concentration of 400 g/lt (230 mmols).

This mixture was brought to 70°C, under vigorous stirring, and was then maintained at that temperature for 16 hours. At the end of this time, the mixture was allowed to stand overnight in a refrigerator (at 0°—5°C).

After filtering the crystals that had thus formed, and after separation of the phases, into the aqueous phase there were first bubbled through $SO_2$, until complete destruction of the residual $H_2O_2$ occurred, and then $N_2$ in order to remove the $SO_2$ in excess.

The aqueous solution was thereupon basified with NaOH in a 10% concentration to a pH of about 8 and was then dried at 60°C under vacuum.

The residue was extracted for 40 minutes with acetone at boiling temperature. The mixture was then filtered and the solid thus obtained was dissolved in water and concentrated to the minimum volume possible. The solution was then acidified with a few drops of concentrated HCl and was then allowed to crystallize in a refrigerator. The crystals thus obtained, added to those gathered previously, were washed with 1,2-dichloroethane, then with icy water (2 ml), and then first dried at a water pump then in an oven for 2 hours at 80°C.

There were obtained 5.43 g of a 99% adipic acid, which corresponded to a yield of 71%.

Example 7

Example 1 was repeated, but using trans-2-octene (2.24 g; 20 mmols) instead of 1-octene.

Both the organic phase at the end of the test as well as the end extract in n-hexane, containing the desired product, were repeatedly shaken with water in order to remove possible traces of residual acetic acid. Thereby were obtained 1.88 g of $C_5$—$C_8$ acids of which 96.3% was capronic ($C_6$) acid. The yield in capronic acid amounted to 78%.

Example 8

Example 4 was repeated, but using 1,2-octanediol (2.92 g; 20 mmols), and using 20 mmols of 1,2-dichloroethane and prolonging the reaction time to 7 hours. At the end of the reaction there was followed the procedure as described in example 1.

There were obtained 2 g of $C_6$—$C_8$ acids of which 96.5% consisted of enanthic ($C_7$) acid. The yield in enanthic acid amounted to 74%.

Example 9

Into a 250 ml flask, fitted with a reflux coolant and a magnetic stirrer, there were loaded 1.4 g (equal to about 2.5 mmols of W) of the composition of example 1, 80 ml of 1,2-dichloroethane, 6 g (51.7 mmols) of 1,2-cyclohexanediol (cis+trans mixture) and 14.45 ml of $H_2O_2$ in a concentration of 400 g/lt (170 mmols).

The mixture was thereupon brought to 70°C, under vigorous stirring, and was then maintained at this temperature for 14 hours. At the end of this time there was followed the procedure as described in example 6. There were obtained 6.15 g of a 95.6% adipic acid, which corresponded to a yield of 78%.

Example 10

Into a 100 ml flask, fitted with a reflux coolant and a magnetic stirrer, there were introduced 1.65 grams of

$$Na_2WO_4 \cdot 2H_2O$$

(5 mmols), 15 ml of $H_2O$, and 1.5 ml of $H_2O_2$ at a concentration of 400 g/litre (17.6 mmols), and then there were admixed $H_2SO_4$ in a 30% concentration until a pH of about 1 was obtained.

To this solution, kept under stirring, there were then added dropwise, in about 2 minutes, 0.8 g (about 2 mmols) of trioctylmethylammonium chloride dissolved in 20 ml of 1,2-dichloroethane. After 15 minutes of further stirring, the organic phase was separated, filtered, and then used as such as indicated in the following.

To the thus obtained solution of 1,2-dichloroethane, containing the catalyst (about 2 mmols of W), there were added 2.92 g (20 mmols) of 1,2-octanediol, 7.65 ml of $H_2O_2$ in a concentration of 400 g/lt (90 mmols), and about 2 mg of p-(terbutyl)phenol.

This mixture was then brought to 80°C, under vigorous stirring, and was then maintained at this temperature for 7 hours. There was then followed the procedure as described in example 1, obtaining 1.78 g of $C_6$—$C_8$ acids of which 96.7% consisted of enanthic ($C_7$) acid. The yield in enanthic acid amounted to 66%.

Example 11

Example 1 was repeated, using 46.75 ml of $H_2O_2$ in a concentration of 80 g/litre (100 mmols). There were obtained 1.95 g of $C_6$—$C_8$ acids of which 90.2% consisted of enanthic ($C_7$) acid. The yield in enanthic acid amounted to 68%.

**Claims**

1. A process for preparing monocarboxylic or bicarboxylic acids by oxidative scission of olefins or vicinal dihydroxy compounds, characterized in that an olefin of formula

$$R_1—CH=CH—R_2 \text{ or } R_1—CH=CH_2$$

or a vicinal dihydroxy compound of formula

$$R_1—CHOH—CHOH—R_2 \text{ or } R_1—CHOH—CH_2OH,$$

wherein $R_1$ and $R_2$ may be the same as or different from each other and are optionally substituted with one or more groups inert under the reaction conditions, and represent hydrocarbon groups such as alkyls having up to 30 carbon atoms; cycloalkyls, optionally branched, having from 3 to 12 carbon atoms; aryls and alkylaryls having from 6 to 12 carbon atoms; and wherein $R_1$ and $R_2$ may be bound to each other so as to form an alkenyl or an alkyl cycle having up to 12 carbon atoms, is reacted, under vigorous stirring, with $H_2O_2$ at a temperature from 0° to 120°C and under a pressure from 1 to 100 atmospheres; in that there are used an aqueous phase containing $H_2O_2$ and an organic phase comprising a solvent, the olefin or the vicinal dihydroxy compound and a catalyst; and in that in the case of an olefin, the catalyst is a composition of the formula:
$Q_3XW_4O_{24-2n}$ wherein:

Q represents an onium $(R_5R_6R_7R_8M)^+$ cation in which M is selected from N, P, As and Sb, and $R_5$, $R_6$, $R_7$ and $R_8$, which may be the same as or different from each other, represent hydrogen atoms or hydrocarbon groups having a total of from 20 to 70 carbon atoms;
X is P or As; and
n is 0, 1 or 2;

and in that in the case of a vicinal dihydroxy compound, the catalyst is selected from said catalysts of composition

$$Q_3XW_4O_{24-2n}$$

and from catalysts obtained by reaction of tungstic acid or an alkali metal tungstate with hydrogen peroxide and an onium $Q^+Y^-$ salt, where Q is as defined above and $Y^-$ is an inorganic anion, at a pH below 2.

2. A process as claimed in claim 1, characterized in that, in the catalyst, the radicals $R_5$, $R_6$, $R_7$ and $R_8$ of the onium $(R_5R_6R_7R_8M)^+$ cation have a total of from 25 to 40 carbon atoms.

3. A process as claimed in claim 1 or 2, characterized in that said catalyst obtained by the reaction of tungstic acid or of an alkali tungstate with hydrogen peroxide and an onium salt is prepared in the reaction medium, by introducing into the said medium (i) tungstic acid or an alkali tungstate or (ii) a tungsten compound capable of forming tungstate ions in the reaction medium, hydrogen peroxide, the onium salt, the vicinal dihydroxy compound, the solvent and, if required, a mineral acid in a quantity sufficient to bring the pH to a value below 2.

4. A process as claimed in claim 3, characterized in that the said tungsten compound capable of forming tungstate ions in the reaction medium is

selected from $WO_2$, $W_2O_5$, $WO_3$, $WS_2$, $WS_3$, $WCl_6$, $WOCl_4$ and $W(CO)_6$.

5. A process as claimed in any of claims 1 to 4, characterized in that the solvent is an aromatic hydrocarbon, a chlorinated hydrocarbon or an alkyl ester.

6. A process as claimed in any of claims 1 to 5, characterized in that the reaction temperature is from 40° to 90°C.

7. A process as claimed in any of claims 1 to 6, characterized in that, in the case of a vicinal dihydroxy compound, the pressure is substantially atmospheric pressure.

8. A process as claimed in any of claims 1 to 7, characterized in that the catalyst is used in an amount of from 0.01 to 1 gram atom of W per mol of olefin or of vicinal dihydroxy compound.

**Patentansprüche**

1. Ein Verfahren zur Herstellung von Monocarbonsäure- oder Bicarbonsäuren durch oxidative Spaltung von Olefinen oder vicinalen Dihydroxyverbindungen, dadurch gekennzeichnet, daß ein Olefin der Formel

$$R_1—CH=CH—R_2 \text{ oder } R_1—CH=CH_2$$

oder eine vicinale Dihydroxyverbindung der Formel

$$R_1—CHOH—CHOH—R_2 \text{ oder } R_1—CHOH—CH_2OH,$$

worin $R_1$ und $R_2$ gleich oder verschieden sein können und gegebenenfalls mit einer oder mehreren, unter den Reaktionsbedingungen inerten Gruppen substituiert sein können und Kohlenwasserstoffgruppen, wie Alkylgruppen mit bis zu 30 Kohlenstoffgruppen; Cycloalkylgruppen, die gegebenenfalls verzweigt sind und von 3 bis 12 Kohlenstoffatome aufweisen; Aryl- und Alkylarylgruppen mit von 6 bis 12 Kohlenstoffatomen darstellen, und worin $R_1$ und $R_2$ unter Bildung eines Alkenyl- oder Alkylringes mit bis zu 12 Kohlenstoffatomen miteinander verbunden sein können, unter heftigem Rühren mit $H_2O_2$ bei einer Temperatur von 0 bis 120°C und unter einem Druck von 1 bis 100 at umgesetzt wird; daß eine wässrige, $H_2O_2$ enthaltende Phase und eine organische Phase, die ein Lösungsmittel, das Olefin oder die vicinale Dihydroxyverbindung und einen Katalysator umfaßt, verwendet werden, und daß im Fall eines Olefins der Katalysator eine Zusammensetzung der Formel

$$Q_3XW_4O_{24-2n}$$

ist, worin

Q ein Onium-$(R_5R_6R_7R_8M)^+$-Kation darstellt, in welchem M ausgewählt ist aus N, P, As und Sb und $R_5$, $R_6$, $R_7$ und $R_8$, die gleich oder verschieden sein können, Wasserstoffatome oder Kohlenwasserstoffgruppen mit insgesamt 20 bis 70 Kohlenstoffatomen bedeuten;

X für P oder As steht, und

n 0, 1 oder 2 ist,

und daß im Fall einer vicinalen Dihydroxyverbindung der Katalysator ausgewählt ist aus Katalysatoren der Zusammensetzung

$$Q_3XW_4O_{24-2n}$$

und aus Katalysatoren, die durch Reaktion von Wolframsäure oder eines Alkalimetall-Wolframats mit Wasserstoffperoxid und einem Onium $Q^+Y^-$ Salz bei einem pH unter 2 erhalten worden sind, wobei Q wie oben definiert ist und $Y^-$ ein anorganisches Anion ist.

2. Verfahren gemäß Anspruch 1, dadurch gekennzeichnet, daß im Katalysator die Gruppen $R_5$, $R_6$, $R_7$ und $R_8$ des

$$\text{Onium-}(R_5R_6R_7R_8M)^+\text{-Kations}$$

insgesamt von 25 bis 40 Kohlenstoffatome aufweisen.

3. Verfahren gemäß Anspruch 1 oder 2, dadurch gekennzeichnet, daß der durch Reaktion von Wolframsäure oder einem Alkaliwolframat mit Wasserstoffperoxid und einem Oniumsalz erhaltene Katalysator in einem Reaktionsmedium hergestellt wird, indem man in dieses Medium (i) Wolframsäure oder ein Alkaliwolframat oder (ii) eine Wolframverbindung, die im Reaktionsmedium Wolframationen bilden kann, Wasserstoffperoxid, das Oniumsalz, die vicinale Dihydroxyverbindung, das Lösungsmittel und, nach Bedarf, eine Mineralsäure in ausreichender Menge, um den pH auf einen Wert unter 2 zu bringen, einführt.

4. Verfahren gemäß Anspruch 3, dadurch gekennzeichnet, daß die Wolframverbindung, die im Reaktionsmedium Wolframationen bilden kann, ausgewählt ist aus $WO_2$, $W_2O_5$, $WO_3$, $WS_2$, $WS_3$ $WCl_6$, $WOCl_4$ und $W(CO)_6$.

5. Verfahren nach irgendeinem der Ansprüche 1 bis 4, dadurch gekennzeichnet, daß das Lösungsmittel ein aromatischer Kohlenwasserstoff, ein chlorierter Kohlenwasserstoff oder ein Alkylester ist.

6. Verfahren nach irgendeinem der Ansprüche 1 bis 5, dadurch gekennzeichnet, daß die Reaktionstemperatur 40 bis 90°C beträgt.

7. Verfahren nach irgendeinem der Ansprüche 1 bis 6, dadurch gekennzeichnet, daß im Fall einer vicinalen Dihydroxyverbindung der Druck im wesentlichen atmosphärischer Druck ist.

8. Verfahren nach irgendeinem der Ansprüche 1 bis 7, dadurch gekennzeichnet, daß der Katalysator in einer Menge von 0,01 bis 1 g-Atom W pro Mol Olefin oder vicinaler Dihydroxyverbindung verwendet wird.

**Revendications**

1. Un procédé de préparation d'acides monocarboxyliques ou dicarboxyliques par coupure oxydante d'oléfines ou de dérivés dihydroxy

vicinaux, caractérisé en ce que l'on fait réagir une oléfine de formules

$R_1—CH=CH—R_2$ ou $R_1—CH=CH_2$

ou un dérivé dihydroxy vicinal de formules

$R_1—CHOH—CHOH—R_2$ ou $R_1—CHOH—CH_2OH$,

dans lesquelles $R_1$ et $R_2$ peuvent être identiques ou différents l'un de l'autre et sont éventuellement substitués par un ou plusieurs groupes inertes dans les conditions de la réaction et représentent des groupes hydrocarbonés tels que des radicaux alkyles renfermant jusqu'à 30 atomes de carbone; cycloalkyles, éventuellement ramifiés, renfermant de 3 à 12 atomes de carbone; aryles et alkylaryles renfermant de 6 à 12 atomes de carbone, et dans lesquelles $R_1$ et $R_2$ peuvent être liés l'un à l'autre de façon à former un cycle alkényle ou un cycle alkyle, renfermant jusqu'à 12 atomes de carbone, sous agitation vigoureuse, avec $H_2O_2$ à une température de 0° à 120°C et sous une pression de 1 à 100 atmosphères; en ce que l'on utilise une phase aqueuse contenant $H_2O_2$ et une phase organique comprenant un solvant, l'oléfine et le composé dihydroxy vicinal et un catalyseur; et en ce que, dans le cas d'une oléfine, le catalyseur est une composition de formule:

$Q_3XW_4O_{24-2n}$

dans laquelle:

Q représente un cation onium $(R_5R_6R_7R_8M)^+$ dans lequel: M est choisi parmi N, P, As et Sb; et $R_5$, $R_6$, $R_7$ et $R_8$, qui peuvent être identiques ou différents l'un de l'autre, représentent des atomes d'hydrogène ou des groupes hydrocarbonés, renfermant un total de 20 à 70 atomes de carbone;
X représente P ou As; et
n est égal à 0, 1 ou 2;

et en ce que, dans le cas d'un dérivé dihydroxy vicinal, le catalyseur est choisi parmi ces catalyseurs de composition

$Q_3XW_4O_{24-2n}$

et parmi des catalyseurs obtenus par réaction de l'acide tungstique ou d'un tungstate de métal alcalin avec le peroxyde d'hydrogène et un sel onium $Q^+Y^-$ dans lequel Q est tel que défini ci-dessus et $Y^-$ représente un anion minéral, à un pH inférieur à 2.

2. Un procédé selon la revendication 1, caractérisé en ce que, dans le catalyseur, les radicaux $R_5$, $R_6$, $R_7$ et $R_8$ du cation onium $(R_5R_6R_7R_8M)^+$ renferment un total de 25 à 40 atomes de carbone.

3. Un procédé selon la revendication 1 ou 2, caractérisé en ce que ce catalyseur obtenu par la réaction de l'acide tungstique ou d'un tungstate alcalin avec le peroxyde d'hydrogène et un sel onium est préparé dans le milieu réactionnel par introduction dans ce milieu (i) d'acide tungstique ou d'un tungstate alcalin ou (ii) d'un dérivé du tungstène capable de donner lieu à la formation d'ions tungstate dans le milieu réactionnel, de peroxyde d'hydrogène, du sel onium, du composé dihydroxy vicinal, du solvant et, si nécessaire, d'un acide minéral en quantité suffisante pour amener le pH à une valeur inférieure à 2.

4. Un procédé selon la revendication 3, caractérisé en ce que ce dérivé du tungstène capable de former les ions tungstate dans le milieu réactionnel est choisi parmi $WO_2$, $W_2O_5$, $WO_3$, $WS_2$, $WS_3$, $WCl_6$, $WOCl_4$ et $W(CO)_6$.

5. Un procédé selon l'une quelconque des revendications 1 à 4, caractérisé en ce que le solvant est un hydrocarbure aromatique, un hydrocarbure chloré ou un alkylester.

6. Un procédé selon l'une quelconque des revendications 1 à 5, caractérisé en ce que la température de réaction est de 40° à 90°C.

7. Un procédé selon l'une quelconque des revendications 1 à 6, caractérisé en ce que que, dans le cas de l'emploi d'un composé dihydroxy vicinal, la pression est sensiblement la pression atmosphérique.

8. Un procédé selon l'une quelconque des revendications 1 à 7, caractérisé en ce que le catalyseur est utilisé en une quantité comprise entre 0,01 et 1 atome-gramme de W par mole d'oléfine ou de composé dihydroxy vicinal.